# EUROPEAN PATENT APPLICATION

(11) **EP 4 116 418 A1**
(43) Date of publication of application: **11.01.2023**
(21) Application number: 21184589.6
(22) Date of filing: 08.07.2021
(51) Int. Cl.: C12N 15/113

(54) **COMPOUND AND METHOD FOR AN ALLELE-SPECIFIC EDITING OF THE ELANE GENE**

(71) Applicant: Eberhard Karls Universität Tübingen Medizinische Fakultät, 72074 Tübingen (DE)
(72) Inventor: Skokowa, Julia, 72127 Kusteringen (DE); Nasri, Masoud, 72074 Tübingen (DE); Mir, Perihan, 70771 Leinfelden-Echterdingen (DE)
(74) Representative: Witte, Weller & Partner Patentanwälte mbB

(57) **Abstract**

The present invention relates to a nucleic acid molecule for an allele-specific editing of the *ELANE* gene, a vector comprising said nucleic acid molecule, a composition comprising said nucleic acid molecule or vector, a method *in vitro* for an allele-specific editing the *ELENE* gene in biological material including genetic material encoding said *ELENE* gene, and to a method for the prophylaxis and/or treatment and/or examination of a disease in a living being.

## Description

The invention is directed to a nucleic acid molecule for an allele-specific editing of the *ELANE* gene, a vector comprising said nucleic acid molecule, a composition comprising said nucleic acid molecule or vector, a method *in vitro* for an allele-specific editing the *ELENE* gene in biological material including genetic material encoding said *ELENE* gene, and to a method for the prophylaxis and/or treatment and/or examination of a disease in a living being.

### FIELD OF THE INVENTION

The present invention relates to the field of molecular medicine, more particular to the field of genetic engineering applications, preferably to the targeted knockdown of disease-associated genes.

### BACKGROUND OF THE INVENTION

A genetic disorder is a health problem caused by one or more abnormalities in the genome. It can be caused by a mutation in a single gene (monogenic) or multiple genes (polygenic) or by a chromosomal abnormality.

A single-gene or monogenic disorder is the result of a single mutated gene. Single-gene disorders can be passed on to subsequent generations in several ways. Genomic imprinting and uniparental disomy, however, may affect inheritance patterns. Single-gene disorders may be categorized as recessive and dominant types as well as autosomal (i.e. non-sex chromosome linked) and X-linked types. Typically, however, it is simply referred to autosomal recessive or autosomal dominant single-gene disorders.

Two copies of the gene (i.e., two alleles) must be mutated for a person to be affected by an autosomal recessive disorder. An affected person usually has unaffected parents who each carry a single copy of the mutated gene and are referred to as genetic carriers. Only one mutated copy of the gene (i.e., one allele) will be necessary for a person to be affected by an autosomal dominant disorder. Each affected person usually has one affected parent. In some cases, there are sporadic mutations that occur for the first time in a child with healthy unaffected parents. Sometimes, one of the parents is mosaic for a dominant mutation, and a child or children are heterozygous for this mutation. A dominant genetic disorder involves a gene or genes which exhibit(s) dominance over a normal (functional/healthy) gene or genes. As such, in dominant genetic disorders only a single copy (i.e., allele) of an abnormal gene is required to cause or contribute to the symptoms of a particular genetic disorder. Such mutations include, for example, gain-of-function mutations in which the altered gene product possesses a new molecular function or a new pattern of gene expression.

Neutropenia is disorder characterized by an abnormally low concentration of neutrophils in the blood and bone marrow. Neutrophils make up the majority of circulating white blood cells and serve as the primary defense against infections by destroying bacteria, bacterial fragments and immunoglobulin-bound viruses in the blood. People with neutropenia are more susceptible to bacterial infections and, without prompt medical attention, the condition may become life-threatening (neutropenic sepsis). Neutropenia can be acute (temporary) or chronic (long lasting). The term is sometimes used interchangeably with "leukopenia" ("deficit in the number of white blood cells").

Neutropenia can be divided into acquired and congenital. The two main types of the congenital condition are severe congenital neutropenia (CN or SCN) and cyclic neutropenia (CyN).

Cyclic neutropenia is characterized by fluctuating neutrophil counts from normal levels to zero while severe congenital neutropenia is characterized by very low absolute neutrophil count (ANC) (500/ml) observed at birth, maturation arrest of the myelopoiesis in bone marrow at the promyelocyte/myelocyte stage, and early onset of bacterial infections.

Severe congenital neutropenia may be diagnosed by measuring a very low ANC in the blood and examining bone marrow aspirate to identify myeloid maturation arrest. It is usually diagnosed shortly after birth, while diagnosis for CyN is generally raised at different ages, and the main clinical manifestation is recurrent acute stomatologic disorders. Bone marrow examination is often necessary to rule out malignant transformation of hematopoiesis, determine cellularity, assess myeloid maturation, and detect signs of a precise etiology, with cytogenetic bone marrow studies now crucial when CN/SCN is suspected. Antineutrophil antibody assay, immunoglobulin assay (Ig GAM), lymphocyte immunophenotyping, pancreatic markers (serum trypsinogen and fecal elastase) and li-posoluble vitamin levels (vitamins A, E and D) are also of interest in assessing CN/SCN and CyN.

Both CN/SCN and CyN are typically autosomal dominant disorders and are mostly caused by heterozygous mutations in the so-called "elastase, neutrophil expressed gene" *(ELANE* gene). However, there are CN/SCN cases with an autosomal recessive inheritance, as for example *HAX1* mutations. The *ELANE* gene encodes neutrophil elastase, a serine proteinase that is involved in the function of neutrophil extracellular traps (NETs, networks of fibers that bind pathogens). Some studies suggest that the product of mutant *ELANE* inhibits maturation of bone marrow hematopoietic stem cells into neutrophils and cause neutropenia. These studies indicate that mutations in the neutrophil elastase initiate the unfolded protein response (UPR) leading to the defective process of neutrophil formation in the marrow. Along with CN/SCN and CyN further *ELANE* gene-associated diseases are known in the art such as emphysema or emphysematous changes.

Currently, neutropenia is treated with the hematopoietic growth factor granulocyte-colony stimulating factor (G-CSF). G-CSF stimulates the production of neutrophils and the delay of their apoptosis. Recombinant G-CSF factor preparations, such as filgrastim, can be effective in people with different forms of neutropenia including severe congenital neutropenia and cyclic neutropenia. The dosage to induce the neutrophil production varies considerably depending on the individual's condition. Overall survival of patients with severe congenital neutropenia is now estimated to exceed 80%, although 10% of CN/SCN patients still die from severe bacterial infections or sepsis. Although G-CSF therapy is successful in preventing mortality from sepsis, long-term treatment was identified to be associated with an increased risk of developing myelodys-plastic syndrome (MDS) or leukemia in CN/SCN patients.

Hematopoietic stem cell transplant (HSCT) is an alternative, curative therapy for patients who do not respond to G-CSF therapy or who develop acute myeloid leukemia or MDS. However, patients with congenital neutropenia who undergo HSCT are at increased risk of developing infectious complications such as fungal and graft-versus-host disease.

In WO 2019/217294 A1 an approach for knocking out the expression of a mutated *ELANE* gene via CRISPR/Cas9 technology is disclosed. However, the disclosed method depends on the existence of single nucleotide polymorphisms (SNPs) which, however, are not prevailing in many mutated *ELANE* genes or which turned out as not being usable for a targeted knockdown.

Against this background it is an object underlying the invention to provide a new approach or compounds which allow a targeted prophylaxis and/or treatment and/or examination of an *ELANE* gene-associated disorder. Furthermore, a compound is to be provided which allows the addressing of a mutated *ELANE* gene in an allele-specific manner. In particular such a compound is to be provided which addresses frequently prevalent leukemia-associated mutations or mutations associated with no response to G-CSF - but not merely rarely occurring polymorphisms - in the *ELANE* gene.

This object is met by the provision of a nucleic acid molecule for an allele-specific editing of the *ELANE* gene comprising a nucleotide sequence which is selected from the group consisting of SEQ ID NOS: 1-3.

The inventors have realized that by using the nucleic acid molecule according to the invention an allele-specific editing of the *ELANE* gene can be achieved. For instance, the nucleic acid molecule comprising or consisting of the nucleotide sequence SEQ ID NO: 1 is configured to specifically target the c.170C>T mutation at exon 2 of human *ELANE* gene or p. A57V mutation of the corresponding gene product, respectively. For instance, the nucleic acid molecule comprising or consisting of the nucleotide sequence SEQ ID NO: 2 is configured to specifically target the c.641G>T mutation at exon 5 of human *ELANE* gene or p. G214V of the corresponding gene product, respectively. According to the findings of the inventors both mutations can frequently be found in autosomal dominant forms of CN/SCN. Due to the specific configuration of the nucleic acid molecule according to the invention a non mutated *ELANE* gene will not be targeted. This specific configuration of the nucleic acid molecule according to the invention, therefore, allows an allele-specific editing of the *ELANE* gene.

As an advantage of this approach, in the presence of a mutated first allele and a non-mutated second allele of the *ELANE* gene only the mutated first allele will be targeted, e.g. knocked-down, while the non-mutated second allele is still capable of expressing the functional *ELANE* gene product. The expression of a mutated *ELANE* gene product with abnormal functions will thereby be inhibited or prevented, respectively. Instead, only the functional *ELANE* gene product prevails. This allows a genetic correction of dominant mutations even without using a repair template.

The inventors were able to demonstrate in an *in vitro* system by using the nucleic acid molecule according to the invention to knockdown the *ELANE* gene, e.g., in form a single guide RNA (sgRNA), that the diminished granulocytic differentiation of hematopoietic stem and progenitor cells (HSPCs) of congenital neutropenia patients could be successfully restored. The restored and differentiated HSPCs showed a significant ROS production in contrast to HSPCs where the *ELANE* gene was not edited by the nucleic acid molecule according to the invention.

The findings of the inventors were surprising and could not be expected.

A 'nucleic acid molecule' as referred to herein includes both deoxyribonucleic acid (DNA) and ribonucleic acid (RNA) molecules, single or double-stranded, comprising concatenated natural and/or chemically modified nucleotides.

According to the invention an *ELANE* gene or 'elastase, neutrophil expressed' gene (human variant: Gene ID: 1991, Ensembl: ENSG00000197561, UniPro-tKB/Swiss-Prot: P08246) encodes a serine protease, also referred to as neutrophil elastase. Neutrophil elastase belongs to the same family as chymotrypsin and has broad substrate specificity. Secreted by neutrophils and macrophages during inflammation, it destroys bacteria and host tissue.

According to the invention 'gene editing' or genomic editing is a type of genetic engineering in which DNA is inserted, deleted, modified or replaced in the genome of a living organism. In the context of the invention 'gene editing' refers to the modification of the *ELANE* gene, e.g. in a biological cell. Therefore, according to the invention 'gene editing' includes the knocking out or knocking down or the correction of the *ELANE* gene, preferably the mutated *ELANE* gene. According to the invention 'allele-specific' means that gene editing specifically targets a mutated allele of the *ELANE* gene while a non-mutated allele remains unaffected. In an embodiment of the invention the gene editing results in a targeted knockdown and a specific functional elimination of the expression of the mutated allele of the *ELANE* gene, respectively. Therefore, the gene-editing according to the invention includes an inactivating in a biological cell of a mutant allele of the *ELANE* gene which cell is heterozygous for said mutation, i.e. said cell further comprises at least one non-mutant allele of the *ELANE* gene.

The problem underlying the invention was herewith fully achieved.

In an embodiment of the invention said nucleic acid molecule is configured for an allele-specific editing of autosomal dominant mutations of the *ELANE* gene.

This measure has the advantage that a significant percentage of *ELANE* gene associated disorders can be specifically addressed. E.g., in CN/SCN patients mutations in the *ELANE* gene are quite often developed in an autosomal dominant manner. A mutation in one of two alleles results in a disease phenotype despite the existence of non-mutated further allele(s). The invention effectively remedies such a genetic constitution.

In another embodiment of the invention the nucleic acid is a 'single guide RNA' (sgRNA).

This measure creates the preconditions for using the nucleic acid molecule according to the invention, especially such comprising or consisting of a nucleotide sequence of SEQ ID NO: 1 (addressing the c.170C>T mutation at exon 2 of human *ELANE* gene or p. A57V mutation of the corresponding gene product) and/or SEQ ID NO: 2 (addressing the c.641G>T mutation at exon 5 of human *ELANE* gene or p. G214V mutation of the corresponding gene product), by the CRISPR/Cas9 method.

According to the invention 'single guide RNA' (sgRNA) or guide RNA is a component of the CRISPR complex which serves to guide the CRISPR endonuclease to its target, e.g., the *ELENE* gene. An sgRNA is a non-coding short ribonucleic acid (RNA) sequence which binds to the complementary target DNA sequence. The sgRNA first binds to the CRISPR endonuclease enzyme and the sgRNA sequence guides the complex via pairing to the specific *ELANE* gene location on the DNA, where the CRISPR endonuclease mediates a double-strand break.

In another embodiment of the invention the nucleic acid molecule is a repair template.

This measure has the advantage that a mutated allele of the *ELANE* gene cannot only be knocked-down, e.g., by using sgRNAs comprising or consisting of a nucleotide sequence of SEQ ID NOs: 1 and/or 2, but can also be repaired, e.g., by a nucleic acid molecule or single stranded oligonucleotide DNA (ssODN) comprising or consisting of a nucleotide sequence of SEQ ID NO: 3. The nucleotide sequence of SEQ ID NO: 3 is derived from exon 5 of the human *ELANE* gene without any mutation. By this measure an additional copy of a functional non-mutated *ELANE* gene or allele is established resulting in an increase of the expression of the gene product.

Therefore, a nucleic acid molecule comprising a nucleotide sequence which is selected from the group consisting of SEQ ID NOS: 1-3 is another subject-matter of the invention.

In another embodiment the nucleic acid molecule of the invention is configured for a use in the prophylaxis and/or treatment and/or examination of a disease, preferably an *ELANE*-associated disease, further preferably of congenital neutropenia, and further preferably of severe congenital neutropenia (CN/SCN), and/or cyclic neutropenia (CyN).

This measure has the advantage that the invention can be used for addressing an *ELANE*-associated disease, in particular CN/SCN or CyN, but also emphysema or emphysematous changes, in a causative manner.

Another subject-matter of the invention relates to a vector comprising the nucleic acid molecule according to the invention.

According to the invention, a 'vector' includes any DNA molecule used as a vehicle to artificially carry a nucleic acid molecule, such as an sgRNA, into another cell, where it can be replicated and/or expressed. Viral vectors are of particular preference as they are characterized by embodying the viruses' specialized molecular mechanisms to efficiently transport genomes inside the cells they infect. The vector may or may not be, in a preferred embodiment of the invention, an adeno-associated vector (AAV).

The features, characteristics, advantages and embodiments described for the nucleic acid molecule according to the invention also apply to the vector according to the invention.

Another subject-matter of the invention relates to a composition comprising the nucleic acid molecule and/or the vector according to the invention.

The features, characteristics, advantages and embodiments described for the nucleic acid molecule and the vector according to the invention also apply to the composition according to the invention.

In an embodiment of the invention said composition further comprises a vector encoding CRISPR associated protein 9 (Cas9), preferably *Staphylococcus aureus* Cas9 (SaCas9).

This measure establishes the requirements for applying the CRISPR/Cas9 technology. Cas9 (CRISPR associated protein 9, formerly called Cas5, Csn1, or Csx12), a 160 kilodalton protein, refers to an enzyme which cleaves the phosphodiester bond within a polynucleotide chain, which is a component of a CRISPR (Clustered Regularly Interspaced Short Palindromic Repeat) complex. The Cas9 is, therefore, capable to specifically bind to the sgRNA which directs the endonuclease to the target nucleic acid. *Staphylococcus aureus* CRISPR Cas9 (SaCas9) is a small Cas9 ortholog and can be packed in AAVs, overcoming their packaging constraints. SaCas9 effective gene targeting has been validated in adult rats. Other smaller Cas9 have been recently discovered, such as originating from *Staphylococcus auricularis* or *Campylobacter jejuni* or *Neisseria meningitidis,* and are likewise suitable.

In an embodiment of the composition according to the invention said Cas9 is under the control of a CAG promoter.

The CAG (CMV enhancer/chicken β-Actin promoter) promoter-driven expression is superior over other promoters, especially the common synapsin's promoter in the long-term. This aspect is crucial in the treatment, prophylaxis or modelling of neurodegenerative disease, which require long-term expression/monitoring. CAG has been the promoter of choice in the latest studies employing AAV.PHP.EB.

In another embodiment of the invention said composition is a pharmaceutical composition comprising a pharmaceutically acceptable carrier.

A "pharmaceutical composition" is a composition suitable for an administration to an animal and/or human being in a medical setting. Preferably, a pharmaceutical composition is sterile and produced according to GMP guidelines.

'Pharmaceutically acceptable carriers' or excipients are well known in the art and include, for example, nanocarriers, nanovectors, aqueous solutions such as water or physiologically buffered saline or other solvents or vehicles such as glycols, glycerol, oils such as olive oil or injectable organic esters. In preferred embodiments, when such pharmaceutical compositions are for human administration, e.g., for parenteral administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as an injection, tablet, capsule (including sprinkle capsule and gelatin capsule), granule, powder, syrup, suppository, or the like. The composition can also be present in a solution suitable for topical administration. Alternatively, the pharmaceutical composition can be present in form of an aerosol administrable by inhalation.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The pharmaceutical composition as well as the methods to be explained below of the present invention may be utilized to treat a living being in need thereof. In certain embodiments, the living being is a mammal such as a human, or another non-human mammal.

In an embodiment to the composition according to the invention the latter is configured for the prophylaxis and/or treatment and/or examination of a disease, preferably of an *ELANE* gene-associated disease, further preferably congenital neutropenia, further preferably severe congenital neutropenia (CN/SCN), and/or cyclic neutropenia (CyN).

Another subject-matter of the invention relates to a method *in vitro* for an allele-specific editing the *ELENE* gene in biological material including genetic material encoding said *ELENE* gene, comprising the step of introducing the nucleic acid molecule and/or said vector and or said composition according to the invention into said biological material, preferably said editing is made via CRISPR/Cas9 technology.

According to the invention, 'biological material including genetic material encoding said *ELANE* gene' includes biological cells, tissues and parts of organisms.

In an embodiment of the invention said biological material comprises hematopoietic stem and progenitor cells (HSPCs).

This measure has the advantage that the *ELANE* gene is edited in such biological material where the mutation is pathologically manifested. E.g. in CN/SCN patients HSPCs show a diminished granulocytic differentiation which, according to the findings of the inventors, can be restored by the nucleic acid molecule according to the invention.

Another subject-matter of the invention relates to a method for the prophylaxis and/or treatment and/or examination of a disease in a living being, e.g. an animal or human being, comprising a step of allele-specific editing the *ELANE* gene in said living being by introducing the nucleic acid molecule and/or said vector and or said composition according to the invention into said living being, preferably said gene editing is made *via* CRISPR/Cas9 technology.

The present invention provides a method for inactivating in a cell a mutant allele of the elastase, neutrophil expressed gene *(ELANE* gene), preferably having a mutation associated with congenital neutropenia, severe congenital neutropenia (CN/SCN) or cyclic neutropenia (CyN), and which *ELANE* gene of said cell is mutated at one ore more nucleotide positions selected from c.170C>T (exon 2) and c.641G>T (exon 5), and/or which *ELANE* gene product of said cell is mutated at one or more amino acid positions selected from p. A57V and p. G214V, the method comprising
- introducing to the cell a composition comprising:
- a CRISPR nuclease or a sequence encoding the CRISPR nuclease; and a first RNA molecule comprising a nucleotide sequence of SEQ ID NO:1 or 2,
wherein a complex of the CRISPR nuclease and the first RNA molecule affects a double strand break in the mutant allele of the *ELANE* gene.

In an embodiment of the said method according to the invention a single stranded oligonucleotide DNA (ssODN) comprising a nucleotide sequence of SEQ ID NO: 3 is additionally introduced into the cell.

The present invention provides for a modified cell obtained by the aforedescribed method of the present invention.

The present invention provides for a method of preparing *in vitro* or ex *vivo* a composition comprising modified cells, the method comprising:
a) isolating or providing HSPCs from cells obtained from a living being, preferably a human being, with an *ELANE* gene mutation related to CN/SCN or CyN and/or suffering from CN/SCN or CyN, and which ELANE gene of said cell is mutated at one ore more nucleotide positions selected from c.170C>T (exon 2) and c.641G>T (exon 5), and/or which *ELANE* gene product of said living being is mutated at one or more amino acid positions selected from p. A57V and p. G214V,
b) introducing to the cells of step (a) a composition comprising:
   a CRISPR nuclease or a sequence encoding the CRISPR nuclease; and a first RNA molecule comprising a nucleotide sequence of SEQ ID NO:1 or 2,
   wherein a complex of the CRISPR nuclease and the first RNA molecule affects a double strand break in the mutant allele of the *ELANE* gene in one or more cells, so as to inactive the mutant allele of the *ELANE* gene in one or more cells thereby obtaining modified cells; optionally
c) culture expanding the modified cells of step (b), wherein the modified cells are capable of engraftment and giving rise to progeny cells after engraftment.

In an embodiment of the said method according to the invention a single stranded oligonucleotide DNA (ssODN) comprising a nucleotide sequence of SEQ ID NO: 3 is additionally introduced into the cell.

In another embodiment of said method according to the invention the latter comprises the additional following step d) where the cells of step (b) or step (c) are administered to a living being for treating the CN/SCN or CyN in the subject.

Therefore, the present invention also provides for a method of treating a living being, preferably a human, afflicted with CN/SCN or CyN, comprising administration of a therapeutically effective amount of the modified cells.

The features, characteristics, advantages and embodiments described for the nucleic acid molecule, vector, and composition according to the invention also apply to the afore-referenced methods according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

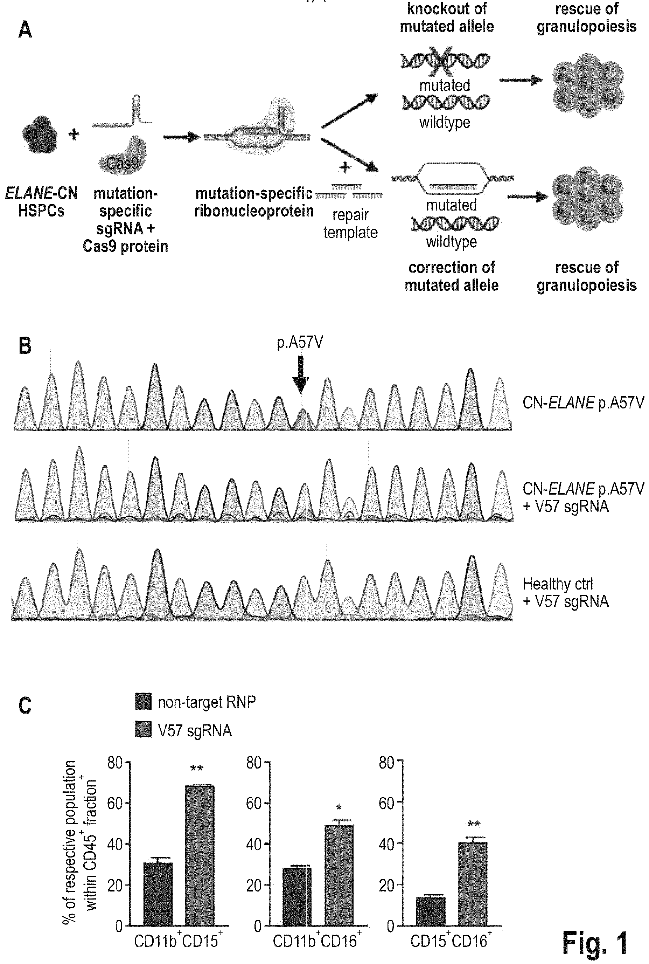

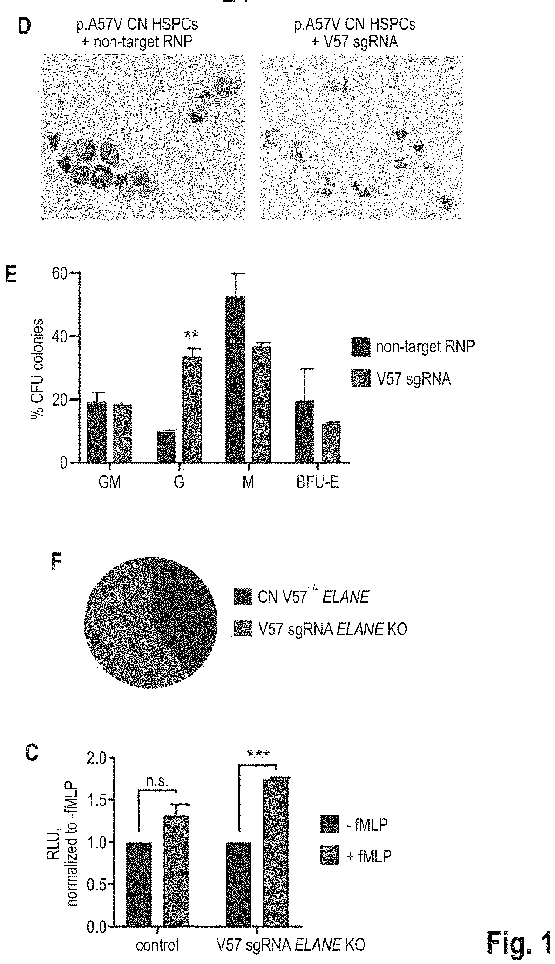

Fig.1: Allele-specific knockout rescues granulopoiesis in p.A57V *ELANE-CN* patient. A, Experimental scheme describing the process of allele-specific gene knockout and allele-specific gene correction. *ELANE-CN* HSPCs were expanded *in vitro* and electroporated with allele-specific Hifi Cas9 RNP. For allele-specific gene correction, a single-stranded DNA repair template (ssODN) was added additionally. After three days cells were seeded for liquid culture differentiation towards neutrophils. Differentiation was assessed on day 14 by flow cytometry and morphologic analysis. B, Gene modification efficiency of V57-specific RNP in *ELANE-CN* and healthy control was analyzed at day 14 of liquid culture differentiation. C, Granulocytic differentiation of *ELANE* V57 knockout CD34⁺ cells was assessed by flow cytometry investigating surface marker expression. D, Representative cytospin images of Wright-Giemsa stained cells 14 days after differentiation. E, Colony-forming unit assay of *ELANE-CN* HSPCs either nucleofected with non-targeting RNP or with mutation-specific RNP. Colonies were counted 14 days after CFU culture. F, CFU-G colonies were picked from mutation-specific KO cells and analyzed by Sanger sequencing. G, Neutrophils from liquid culture differentiation were investigated for their function to generate ROS after treatment with fMLP.

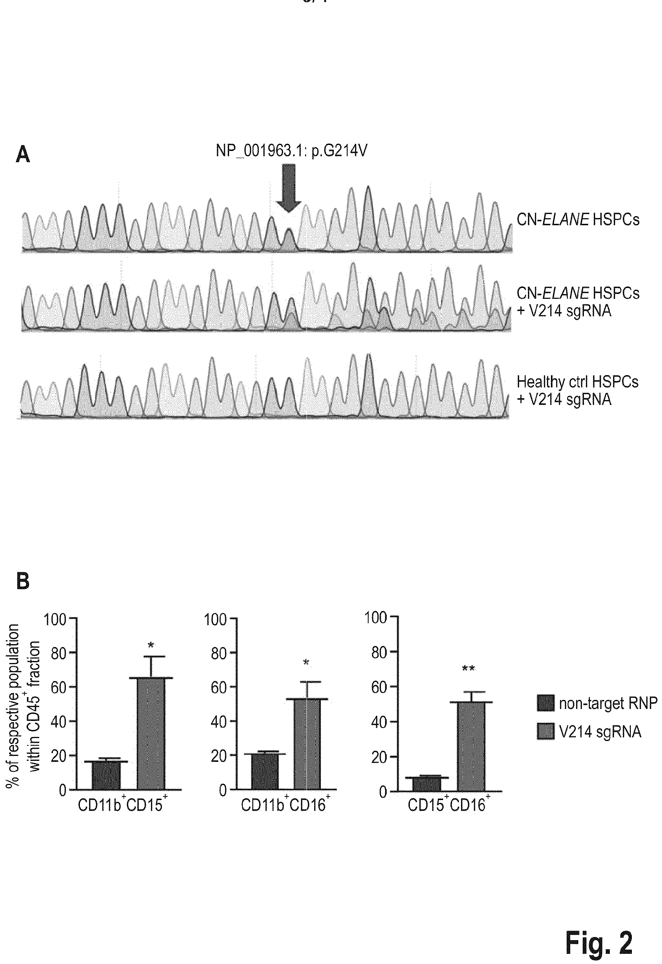

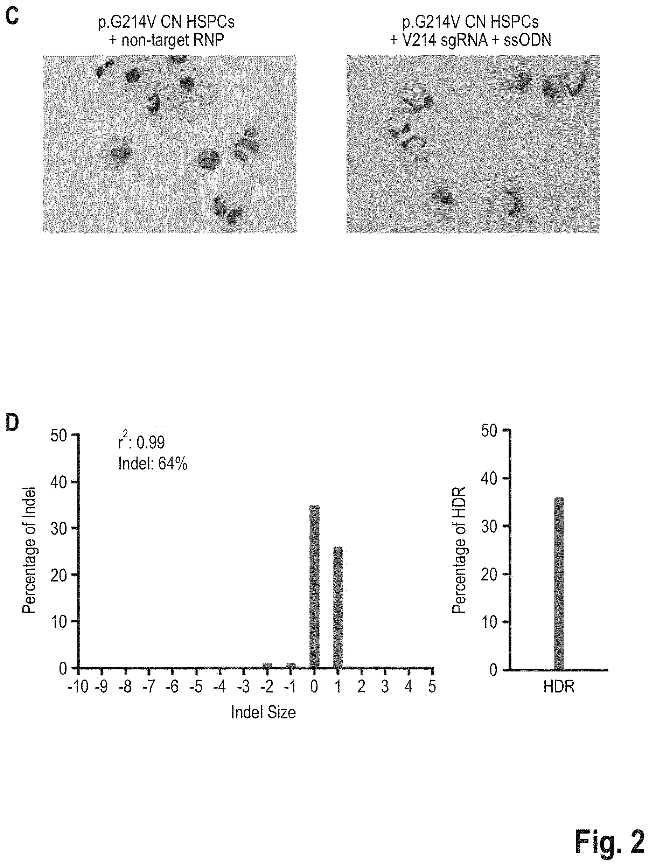

Fig. 2: Allele-specific gene correction of p.G214V *ELANE-CN* patient improved granulocytic differentiation compared to unedited control cells. A, Liquid culture differentiation of p.G214V ELANE-CN HSPCs nucleofected either with non-target RNP or with V214-specific RNP and ssODN correction template was performed. Surface marker expression was investigated after 14 days. B, Representative cytospin images of Wright-Giemsa stained cells 14 days after differentiation C, Allele-specific gene editing efficiency of p.G214V assessed by Sanger sequencing and sequence trace decomposition algorithm (TIDE) revealed 64 % indels. HDR percentage is around 36 %. R2 is calculated to assess the goodness of fit by ICE algorithm and R2 > 0.9 is considered a reliable prediction.

### EXAMPLES

### 1. Allele-specific design strategy and validation of sgRNA targeting mutated ELANE

The inventors generated allele-specific single-guide RNA (sgRNA) that selectively target mutations of the *ELANE* gene (Table 1). p. A57V mutation specific sgRNA for the knockout of the mutated allele of ELANE gene (cut site: chr19 [+852,969: - 852,969], NM_001972.3 Exon 2, 161 bp; NP_001963.1 p.F54) was designed using the CCTop website and synthesized as chemically modified sgRNA by Integrated DNA technologies (IDT). p. G214V mutation specific sgRNA for the selective targeting of the mutated allele of ELANE gene (cut site: chr19 [+856,001: -856,001], NM_001972.3 Exon 5, 641 bp; NP_001963.1 p.V214) was designed using the CCTop website and synthesized as chemically modified sgRNA by Integrated DNA technologies (IDT).

V57 sgRNA was incubated with recombinant HiFi-Cas9 protein to generate CRISPR/Cas9-gRNA RNP complex. The V57 sgRNA targeting mutated exon 2 of *ELANE* was selected to introduce stop-codon mutations and to induce nonsense-mediated mRNA decay (NMD) of ELANE mutated mRNA only, which is caused by stop-codon or frameshift mutations at the beginning of *ELANE* mRNA of mutated allele which hypothetically restore the impaired granulopoiesis in CN/SCN patients (Fig. 1A). To evaluate allele-selectivity of V57 sgRNA the RNP complex of HiFi Cas9 - V57 sgRNA electroporated on CN/SCN patient's HSPCs with p.A57V mutation and healthy control HSPC. The inventors' results show the V57 sgRNA has high selective on-target activity on mutated-allele as the Sanger sequencing results performed 96-hour post electroporation. The V57 mutation-specific sgRNA introduced no DSB on healthy control HSPCs (Fig. 1B) which is another proof for allele-specificity of V57 sgRNA.

**Table 1: sgRNAs and ssODN sequences**

| Name | Sequence | SEQ ID NO: |
|---|---|---|
| V57 sgRNA | 5' TCAGGGTGACGCCGCAGAAG 3' | 1 |
| V214 sgRNA | 5' GGACGAAGGAGGCAATTACG 3' | 2 |
| ssODN repair template for G214V mutation | 5' AGAGTCGATCCAGTTTACAAACTGTGCCACCGG GGCAAAGGCATCGGGGTAGAGCCCTGAGGCGCA GCCTCCCCGGACGAAGGAGGCAATTCCGTGGATT AGCCCGTTGCAGACCAAGGGGCTGCCGGAGTCC CCCTGTGGAGGCAGACAAGGTGGGGAAGCTGGA CTGCGAGGCCCTGCCCA 3' | 3 |

To further evaluate the applicability of CRISPR/Cas9-based allele-specific design strategy on a wide spectrum of *ELANE* mutations, the inventors designed sgRNA for p.G214V mutation at exon 5 of *ELANE* - named V214 sgRNA- (cut site: chr19 [+856,002: -856,002]). The HiFi Cas9 - V214 sgRNA complex along with single-stranded oligonucleotide DNA (ssODN) (Table 1) based repair template electroporated on CN/SCN patient's HSPCs with p. G214V. The Sanger sequencing analysis showed a high level of allele-selectivity of R214 sgRNA. The R214 mutation-specific sgRNA introduced no DSB on healthy control HSPCs (Fig. 2A).

These results confirm the applicability of an allele-specific CRISPR/Cas9 based gene editing for *ELANE*-related congenital neutropenia.

### 2. ELANE knockout in HSPC of ELANE-CN/SCN patients restores diminished granulocytic differentiation

To further evaluate the clinical applicability of allele-specific ELANE knockout as a therapeutic opportunity of ELANE-associated CN/SCN, the inventors used V57 sgRNA to performed CRISPR/Cas9 RNP-mediated gene editing in primary bone marrow CD34⁺ HSPC of ELANE-CN/SCN patient with p.A57V mutation and differentiated the cells towards neutrophils in vitro. Allele-specific *ELANE* knockout in CD34⁺ HSPC was performed by electroporation of human CD34⁺ HSPC with assembled V57 sgRNA and HiFi-Cas9 protein. Allele-specific *ELANE* knockout leads to elevated granulocytic differentiation, as assessed by the percentage of CD15⁺CD11b⁺CD45⁺, CD16⁺CD11b⁺CD45⁺ and CD15⁺CD16⁺CD45⁺ cells (Fig. 1C) and morphological examination of cytospin preparations of mature granulocytes generated on day 14 of the *in vitro* granulocytic differentiation using liquid culture (Fig. 1D). Also, CFU assay was performed with CD34⁺ cells from allele-specific knockout CN/SCN patients showed elevated levels of CFU-G but reduced CFU-M colony numbers, as compared to CD34⁺ cells derived from non-target RNP MOCK treated CN/SCN HSPCs which is electroporated with a sgRNA targeting nowhere in the human genome assembled with HiFi-Cas9 (Fig. 1E). These data suggest that allele-specific ELANE knockout restores granulocytic differentiation in CN/SCN. The inventors picked CFU-G colonies from the CFU assay and genotyped the editing outcome by Sanger sequencing of *ELANE* exon 2. The inventors could show that 66.67% of CFU-Gs were generated by allele-specific *ELANE* knockout HSPCs, while 13.33% of CFU-Gs were generated from HSPCs with spontaneous correction and 20% were un-edited (Fig. 1F).

### 3. Neutrophils generated from ELANE KO HSPC exhibited unaffected ROS production upon activation in vitro

The inventors further evaluated *in vitro* activation of neutrophils generated from allele-specific *ELANE* knockout HSPCs in liquid culture for 14 days. The inventors assessed H₂O₂ levels (ROS) in fMLP-activated allele-specific ELANE knockout neutrophils. They detected very significant ROS production in allele-specific knockout neutrophils after activation with fMLP while the non-target RNP MOCK neutrophils could not produce a significant response after fMLP activation (Fig. 1G).

### 4. ELANE correction in HSPC of p.G214V ELANE-CN/SCN restores diminished granulocytic differentiation.

For allele-specific *ELANE* correction, the inventors used V214 sgRNA to perform CRISPR/Cas9 RNP-mediated gene correction in primary bone marrow CD34⁺ HSPC of *ELANE*-CN/SCN patient with p.G214V mutation and differentiated the cells towards neutrophils *in vitro.* Allele-specific *ELANE* correction in CD34⁺ HSPC was performed by electroporation of human CD34⁺ HSPC with assembled V214 sgRNA and HiFi Cas9 protein along with ssODN as repair template. The V214 mutation-specific sgRNA introduced no DSB on healthy control HSPCs (Fig. 2A). Allele-specific *ELANE* correction leads to elevated granulocytic differentiation, as assessed by the percentage of CD45⁺CD15⁺CD11b⁺, CD45⁺CD11b⁺CD16⁺ and CD45⁺CD15⁺CD16⁺ cells (Fig. 2B). Also, morphological examination of cytospin preparations of mature granulocytes generated on day 14 of the *in vitro* granulocytic differentiation showed increased numbers of neutrophils in the *ELANE* corrected cells compared to control cells (Fig. 2C). These data demonstrate that allele-specific *ELANE* correction restores granulocytic differentiation in CN/SCN. The inventors evaluated the allele-specific mutation correction outcome by performing Sanger sequencing and analyzed the traces by ICE algorithm revealing indel efficiency of 64 % and HDR of 36 % (Fig. 2D).

### 5. Conclusion

The inventors impressively demonstrated that by using the nucleic acid molecule according to the invention, in particular such molecule comprising a nucleotide sequence of SEQ ID NO: 1 and/or 2, the ELANE gene can be edited in an allele-specific manner. This allele-specific editing of the *ELANE* gene is, e.g., realized *via*

## Claims

1. A nucleic acid molecule for an allele-specific editing of the *ELANE* gene comprising a nucleotide sequence which is selected from the group consisting of SEQ ID NOS: 1-3.

2. The nucleic acid molecule of claim 1, which is configured for an allele-specific editing of autosomal dominant mutations of the *ELANE* gene.

3. The nucleic acid molecule of claim 1 or 2 which is a 'single guide RNA' (sgRNA).

4. The nucleic acid molecule of claim 1 or 2 which is a repair template.

5. The nucleic acid molecule of any of claims 1-4 for use in the prophylaxis and/or treatment and/or examination of a disease.

6. The nucleic acid molecule of claim 6, **characterized in that** said disease is congenital neutropenia, preferably severe congenital neutropenia (CN/SCN), and/or cyclic neutropenia (CyN).

7. A vector comprising the nucleic acid molecule of any of claims 1-6.

8. A composition comprising the nucleic acid molecule of any of claims 1-6 and/or the vector of claim 7.

9. The composition of claim 8, further comprising a vector encoding CRISPR associated protein 9 (Cas9), preferably *Staphylococcus aureus* CRISPR Cas9 (SaCas9), further preferably said Cas9 is under the control of a CAG promoter.

10. The composition of claim 8 or 9, which is a pharmaceutical composition comprising a pharmaceutically acceptable carrier.

11. The composition of any of claims 8-10 for the prophylaxis and/or treatment and/or examination of a disease, preferably of congenital neutropenia, further preferably severe congenital neutropenia (CN/SCN), and/or cyclic neutropenia (CyN).

12. A method *in vitro* for an allele-specific editing of the *ELENE* gene in biological material including genetic material encoding said *ELENE* gene, comprising the step of introducing the nucleic acid molecule of any of claims 1-6, and/or said vector of claim 7, and or said composition of any of claims 8-11 into said biological material, preferably said editing is made *via* CRISPR/Cas9 technology.

13. The method of claim 12, **characterized in that** said biological material comprises hematopoietic stem and progenitor cells (HSPCs).

14. A method for the prophylaxis and/or treatment and/or examination of a disease in a living being, comprising a step of allele-specific editing of the *ELANE* gene in said living being by introducing the nucleic acid molecule of any of claims 1-6, and/or said vector of claim 7, and or said composition of any of claims 8-11 into said living being, preferably said gene editing is made via CRISPR/Cas9 technology.

15. The method of claim 13, **characterized in that** said disease is congenital neutropenia, preferably severe congenital neutropenia (CN/SCN), and/or cyclic neutropenia (CyN).
